# EUROPEAN PATENT SPECIFICATION

(11) **EP 4 218 612 B1**
(45) Date of publication and mention of the grant of the patent: **07.10.2026**
(21) Application number: 22871146.1
(22) Date of filing: 12.07.2022
(51) Int. Cl.: A61B 17/072, A61B 17/00, A61B 17/29

(54) **ELECTRIC ANASTOMOSIS DEVICE**
ELEKTRISCHE ANASTOMOSEVORRICHTUNG
DISPOSITIF D'ANASTOMOSE ÉLECTRIQUE

(30) Priority: 15.12.2021 CN 202111536645
(43) Date of publication of application: 02.08.2023
(73) Proprietor: NINGBO HITCM MEDICAL DEVICES CO., LTD., Ningbo, Zhejiang 315000 (CN)
(72) Inventor: LU, Shaolei, Ningbo Zhejiang 315000 (CN); ZHANG, Zhixing, Ningbo Zhejiang 315000 (CN)
(74) Representative: Viering, Jentschura & Partner mbB Patent- und Rechtsanwälte
(86) International application number: PCT/CN2022/105177
(87) International publication number: WO 2023/109109

(56) References cited:
- CN-A- 110 840 501
- CN-A- 111 803 164
- CN-A- 111 870 319
- CN-A- 111 870 319
- CN-A- 112 472 175
- CN-A- 112 472 175
- CN-U- 217 162 191
- CN-U- 217 285 915
- US-A1- 2014 014 707
- US-A1- 2018 168 677

## Description

### TECHNICAL FIELD

The present disclosure relates to a surgical instrument, and more specifically, to an electric stapling instrument.

### BACKGROUND

Electric stapling instrument is a kind of surgical instrument, which can be used to replace traditional manual suture equipment. It has the advantages of fast suture, simple operation as well as few side effects and surgical complications. An electric stapling instrument usually includes an end effector configured for transition from an open configuration to a closed configuration, and a cutter assembly that is movable in a distal direction or a proximal direction. Existing electric stapling instruments involve defects such as unreasonable design for driving device, larger size and weight, higher cost and the like. To this end, the present disclosure proposes an electric stapling instrument having a novel driving device.

CN111870319A discloses a driving device, a surgical instrument and an operating method of the surgical instrument. The driving device is driven by a power assembly and comprises a first driving assembly and a first movement transforming assembly, wherein the first driving assembly comprises a first effective rotational stroke structure and a first idle stroke structure; and the driving device has a first state and a second state, in the first state, the power assembly and the first effective rotational stroke structure are jointed, the first effective rotational stroke structure drives the first movement transforming assembly, in the second state, the power assembly and the first idle stroke structure are coupled, and the first movement transforming assembly is disengaged from the driving of the power assembly. In the second state, the first movement transforming assembly is disengaged from driving of the power assembly, reverse acting force borne by the movement transforming assembly cannot be transmitted to the power assembly through the first driving assembly, and therefore, the driving efficiency of the driving device is prevented from being affected.

CN112472175A discloses a surgical instrument, which comprises an end effector, a driving device for driving the end effector to be opened and closed, and an opening device, the surgical instrument has two states, in the first state, the driving device drives the end effector to be opened or closed, and in the second state, the opening device drives the end effector to be opened, the driving device comprises a driving part, a transmission part and a motion conversion mechanism arranged between the driving part and the transmission part, the surgical instrument further comprises a clutch piece, the transmission part comprises a first part and a second part, and in the first state, the clutch piece enables the first part and the second part to be in transmission connection, and in the second state, the first part and the second part are separated from transmission connection. When a motor and related mechanical and electronic components break down, the end effector can be opened easily and conveniently, medical negligence is avoided, and follow-up operations can be carried out smoothly and conveniently.

### SUMMARY

The invention is defined in independent claim 1. Certain optional features of the invention are defined in the dependent claims. The present disclosure provides an electric stapling instrument.

An electric stapling instrument is characterized by including: a motor assembly including an output shaft configured to rotate in a first direction or a second direction; a transmission mechanism including a driving gear and a first driving assembly, the driving gear being installed on the output shaft of the motor assembly and being configured to rotate with the output shaft, wherein the first driving assembly includes: a first gear engaged with the driving gear and configured to rotate with the driving gear, a second gear coaxially arranged with the first gear, the second gear being at least partially engaged with the driving gear and configured to rotate with the driving gear, and a push rod movably connected with the second gear and configured to move in a proximal direction or a distal direction along with a rotation of the second gear; a sleeve assembly having a proximal end connected with the push rod, the sleeve assembly being configured to move in the proximal direction or the distal direction along with the push rod; and an end effector arranged at a distal end of the sleeve assembly, the end effector including an anvil and a staple cartridge, and at least one of the anvil and the staple cartridge is configured to be pivotable between an open position and a closed position; the second gear includes a second boss, and the push rod includes an elongated hole configured to receive the second boss, wherein the second boss is configured to move between a third position and a fourth position in the elongated hole so that the push rod moves in the proximal direction or the distal direction along with a rotation of the second gear.

In some embodiments, the first gear may include a first boss and the second gear may include a groove configured to receive the first boss, wherein the first boss is configured to move between a first position and a second position in the groove, so that the push rod moves in the proximal direction or the distal direction along with a rotation of the second gear.

In some embodiments, the transmission mechanism may further include a second driving assembly including: a third gear engaged with the driving gear and configured to rotate with the driving gear; a fourth gear engaged with the third gear and configured to rotate with the third gear; and a rack engaged with the fourth gear and configured to move in the proximal direction or the distal direction along with a rotation of the fourth gear. The electric stapling instrument may further include a cutter assembly and a central spindle arranged in the sleeve assembly, wherein a proximal end of the central spindle is connected with the rack, and the central spindle is configured to move in the proximal direction or the distal direction along with the rack; a proximal end of the cutter assembly is connected with a distal end of the central spindle, and the cutter assembly is configured to move in the proximal direction or the distal direction along with the central spindle..

In some embodiments, the anvil and the staple cartridge can be connected with the distal end of the sleeve assembly, respectively, and at least one of the anvil and the staple cartridge can be configured to be pivotable between an open position and a closed position. When the sleeve assembly moves in the proximal direction, the anvil or the staple cartridge pivots to the open position; and when the sleeve assembly moves in the distal direction, at least one of the anvil and the staple cartridge pivots to the closed position.

In some embodiments, when the output shaft of the motor assembly rotates in the first direction and drives the driving gear to rotate in the first direction, both the first gear and the second gear can rotate in the second direction along with the driving gear, so that the second boss moves from the third position to the fourth position in the elongated hole, causing the push rod to move in the distal direction, and in turn causing the sleeve assembly to move in the distal direction, thereby allowing at least one of the anvil and the staple cartridge to pivot from the open position to the closed position. In some embodiments, when the output shaft of the motor assembly rotates in the second direction and drives the driving gear to rotate in the second direction, both the first gear and the second gear can rotate in the first direction along with the driving gear, so that the second boss moves from the fourth position to the third position in the elongated hole, causing the push rod to move in the proximal direction, and in turn causing sleeve assembly to move in the proximal direction, thereby allowing at least one of the anvil and the staple cartridge to pivot from the closed position to the open position.

In some embodiments, the second gear may further include a toothless portion and a toothed portion, and a distal end of the electric stapling instrument may be further provided with a cutter assembly which is connected with the transmission mechanism and configured to move in the proximal direction or the distal direction. Moreover, when the cutter assembly moves in the proximal direction or the distal direction, the first driving assembly may be further configured such that: when the first gear rotates, the first boss moves from the first position to the second position or moves from the second position to the first position in the groove, so that the first gear idles relative to the second gear without driving the second gear to rotate; and the toothless portion of the second gear is joined with the driving gear so as not to rotate the second gear with the driving gear, so that the sleeve assembly remains motionless, thereby remaining at least one of the anvil and the staple cartridge in the closed position or the open position.

In some embodiments, the electric stapling instrument may further include a first limit switch arranged on a movement path of the sleeve assembly; the motor assembly is configured to stop rotating when the sleeve assembly traveling in the distal direction contacts and triggers the first limit switch, so that at least one of the anvil and the staple cartridge is in the closed position. In some embodiments, the electric stapling instrument may further include a second limit switch arranged on the movement path of the sleeve assembly; the motor assembly is configured to stop rotating when the sleeve assembly travelling in the proximal direction contacts and triggers the second limit switch, so that at least one of the anvil and the staple cartridge is in the open position.

In some embodiments, the electric stapling instrument may further include a third limit switch arranged on a movement path of the rack; the motor assembly is configured to stop rotating when the rack travelling in the distal direction contacts and triggers the third limit switch, so that the cutter assembly is in place by moving in the distal direction.

### BRIEF DESCRIPTION OF DRAWINGS

Hereinafter, embodiments of the present disclosure will be further described by way of example with reference to the accompanying drawings which constitute a part of this specification. In the drawings:
Fig. 1 is a perspective view of an electric stapling instrument according to an exemplary embodiment, with some components removed to illustrate internal structure and components, in which an end effector, a sleeve assembly and a motor assembly are illustrated;
Fig. 2 is another perspective view of an electric stapling instrument according to an exemplary embodiment, with some components further removed to illustrate a transmission mechanism as well as a first driving assembly and a second driving assembly thereof according to the exemplary embodiment in details, in which a central spindle is also illustrated;
Fig. 3 is yet another perspective view of an electric stapling instrument according to an exemplary embodiment, in which a transmission mechanism as well as a first driving assembly and a second driving assembly thereof according to the exemplary embodiment are illustrated in details, structures and connection relationships of a third gear and a fourth gear are especially further illustrated, and a cutter assembly is also illustrated;
Fig. 4 is an enlarged view of a circled part in Fig. 1, illustrating an actuator and a third limit switch according to an exemplary embodiment;
Fig. 5 is a perspective view of a first gear and a second gear combined together according to an exemplary embodiment, illustrating a second boss of the second gear;
Fig. 6 is a perspective view of a first gear according to an exemplary embodiment, illustrating a first boss of the first gear;
Fig. 7 is a perspective view of a second gear according to an exemplary embodiment, illustrating a toothed portion, a toothless portion and a groove of the second gear;
Fig. 8 is a perspective view of a push rod according to an exemplary embodiment, illustrating a round hole and an elongated hole of the push rod; and
Fig. 9 is a perspective view of a third gear according to an exemplary embodiment, illustrating a first toothed portion, a second toothed portion and a toothless portion of the third gear.

### DETAILED DESCRIPTION

Further features, advantages and technical effects of the present disclosure will be more clearly and thoroughly understood for those skilled in the art from the following detailed description of the embodiments of the present disclosure in conjunction with the accompanying drawings and specific implementations. In the following description, spatial and orientation terms such as "upper", "lower", "front", "rear", "top", "bottom", "vertical" and "horizontal" may be used to describe the embodiments of the present disclosure, but it should be understood that these terms are only for the convenience of explaining the embodiments illustrated in the drawings and are not intended to require a device to be actually constructed or operated in a specific orientation. In the following description, the use of terms such as "connected", "coupled", "fixed" and "attached" may refer to two elements or structures being directly connected without other elements or structures, or two elements or structures being indirectly connected through intervening elements or structures, unless explicitly stated otherwise herein. The use of ordinal numbers, such as "first" and "second", is only for distinguishing the cited elements from each other, and has no significance in their order or importance unless explicitly stated otherwise herein. As used herein, the terms "proximal/proximal end" and "distal/distal end" are defined relative to a clinician who manipulates a handle portion of a surgical instrument. The term "proximal/proximal end" refers to a part of an involved component or structure that is closer to the clinician, and the term "distal/distal end" refers to a part of the involved component or structure that is away from the clinician.

Existing electric stapling instruments involve defects such as unreasonable design for driving device, large size and weight, high cost and the like. Therefore, the present disclosure provides an electric stapling instrument with a novel driving device.

### I. Exemplary Structure of Electric Stapling Instrument

First of all, the structure of an electric stapling instrument according to an exemplary embodiment will be described with reference to the accompanying drawings.

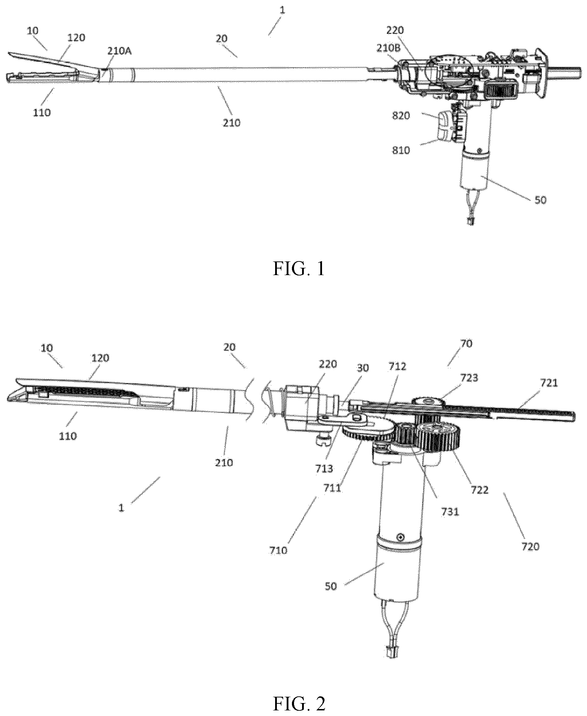

Fig. 1 is a perspective view of an electric stapling instrument 1 according to an exemplary embodiment, with some components of the electric stapling instrument 1 removed to illustrate the internal structures and components. As illustrated in Fig. 1, the electric stapling instrument 1 includes an end effector 10 for performing a stapling operation or the like on a patient. The end effector 10 includes a staple cartridge 110 for receiving staples, and an anvil 120 having an end pivotally connected to the staple cartridge 110. At this end, the anvil 120 is pivotable about the staple cartridge 110 between a closed position and an open position, thereby closing and opening the end effector 10. In some other embodiments, the staple cartridge 110 can also be configured to pivot relative to the anvil 120. However, hereinafter only the embodiment in which the anvil 120 pivots relative to the staple cartridge 110 will be described by way of example, for convenience of explanation.

Fig. 1 also illustrates a sleeve assembly 20. The sleeve assembly 20 includes a tubular-shaped sleeve 210, and a sleeve holder 220 configured to drive the sleeve 210 to move in a proximal direction and a distal direction. A distal end portion 210A of the sleeve 210 is connected to the end effector 10, and a proximal end portion 210B thereof is connected to the sleeve holder 220 so as to be driven by the sleeve holder 220. When the sleeve assembly 20 moves in the distal direction, i.e., when the sleeve 210 is driven to move in the distal direction by the sleeve holder 220, the distal end portion 210A of the sleeve 210 will act on the anvil 120 so that the anvil 102 pivots towards the closed position. When the sleeve assembly 20 moves in the proximal direction, i.e., when the sleeve is driven to move in the proximal direction by the sleeve holder 220, the distal end portion 210A of the sleeve 210 leaves the anvil 120, so that the anvil 120 pivots towards the open position. The working principle of driving the sleeve assembly 20 to move in the proximal direction and the distal direction will be described in further details below.

Furthermore, a motor assembly 50 of the electric stapling instrument 1 and buttons 810 and 820 for operating the electric stapling instrument 1 are also illustrated at the right side of Fig. 1. The motor assembly 50 is rotatable in a first direction (for example, the clockwise direction based on the electric stapling instrument 1 illustrated in Fig. 1 in this embodiment) and in a second direction (the counterclockwise direction) opposite to the first direction to provide power to realize the corresponding functions of the electric stapling instrument. It should be noted that the positions and forms of the motor assembly 50 and the buttons 810, 820 are not limited to the illustrated embodiment, but other alternative forms can be envisaged. For example, the motor assembly 50 may be arranged in other positions, or the buttons may be replaced by other switches such as triggers and knobs.

Now turning to Fig. 2, which is another perspective view of an electric stapling instrument 1 according to an exemplary embodiment. In addition to the end effector 10 and the sleeve assembly 20 already illustrated in Fig. 1, Fig. 2 further illustrates a transmission mechanism 70 and a central spindle 30. The central spindle 30 is arranged in the sleeve assembly 20, and the central spindle 30 has a proximal end connected to the driving mechanism 70 and a distal end connected to a cutter assembly (to be described later). The central spindle 30 can be driven by the transmission mechanism 70 so as to drive the cutter assembly to move in the proximal direction and the distal direction.

The transmission mechanism 70 includes a driving gear 731, a first driving assembly 710 and a second driving assembly 720. The driving gear 731 is fixed on an output shaft of the motor assembly 50, so that the driving gear 731 can be driven by the output shaft of the motor assembly 50 to rotate in the first direction and the second direction. The first driving assembly 710 is configured to drive the anvil 120 to be opened and closed. The second driving assembly 720 is configured to drive the cutter assembly to move in the proximal direction and the distal direction. The structures of the first driving assembly 710 and the second driving assembly 720 will be described in more details below.

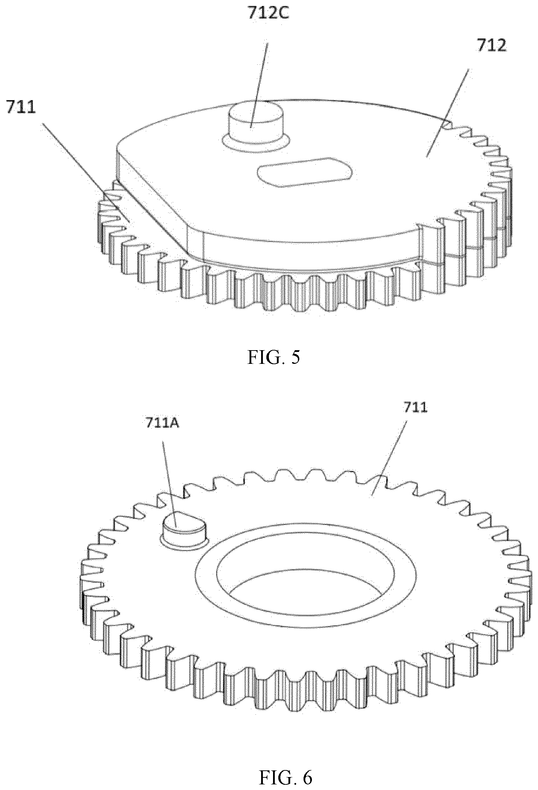

Now still referring to Fig. 2, in conjunction with Figs. 5-8, which illustrate further details of the components included in the first driving assembly 710. The first driving assembly 710 includes a first gear 711, a second gear 712 and a push rod 713.

As illustrated in Figs. 2 and 5, the first gear 711 and the second gear 712 are coaxially arranged. As best illustrated in Fig. 6, the first gear 711 is a full-toothed gear which is engaged with the driving gear 731. Fig. 6 also illustrates that the first gear 711 further includes a first boss711A.

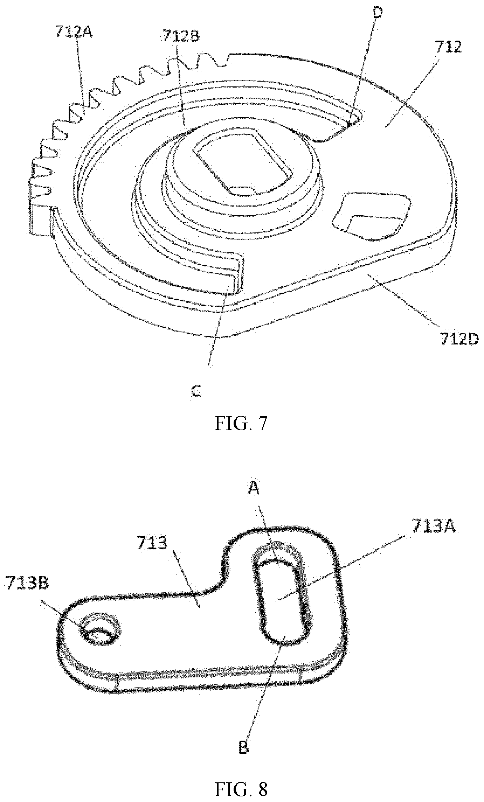

As best illustrated in Fig. 7, the second gear 712 is not a full-toothed gear, and the second gear 712 includes a toothed portion 712A and a toothless portion 712D. As a result, the second gear 712 is engaged with the driving gear 731 only when the toothed portion 712A is rotated to a position where it's engaged with the driving gear 731. Fig. 7 also illustrates that the second gear 712 further includes an arc-shaped groove 712B and a second boss 712C. The first boss711A of the first gear 711 is slidable between a position C (i.e., the first position) and a position D (i.e., the second position) in the arc-shaped groove 712B of the second gear 712. The first boss711A of the first gear, and the arc-shaped groove 712B and the toothless portion 712D of the second gear are cooperated with each other to constitute an idling mechanism, the specific principle of which will be described in further details below.

As illustrated in Fig. 8, the push rod 713 includes a hole 713A. The push rod 713 has one end provided with a circular hole 713B for fixing with the sleeve holder 220, and the other end provided with an elongated hole 713A in which the second boss 712C of the second gear 712 is slidable between a position A and a position B.

In the embodiment of the present disclosure, during a movement of the second boss 712C from the position A to the position B in the elongated hole 713A, the push rod 713 will drive the sleeve holder 220 to move in the distal direction, thus driving the sleeve 210 to move in the distal direction, so that the distal end portion 210A of the sleeve 210 drives the anvil 120 to pivot to a closed position. In the opposite case, during a movement of the second boss 712C from the position B (i.e., the fourth position) to the position A (i.e., the third position) in the elongated hole 713A, the push rod 713 will drive the sleeve holder 220 to move in the proximal direction, thus driving the sleeve 210 to move in the proximal direction, so that the distal end portion 210A of the sleeve 210 will leave the anvil 120, and then the anvil 120 pivots to an open position.

It should be noted that during the closing operation, i.e., during the movement of the second boss 712C from the position A to the position B, a friction force between the second boss 712C and the elongated hole 713A will be gradually decreased. The specific principle is as follows: in the existing driving mechanism, the boss is arranged on a connecting rod, and the elongated hole is arranged in the gear, so that when the elongated hole pushes the connecting rod to move, the connecting rod only moves forwards and backwards, i.e., the boss on the connecting rod always has only one face rubbed against the elongated hole, i.e., a sliding friction is formed; by arranging the second boss 712C on the second gear 712 and arranging the elongated hole 713A in the push rod 713, the second boss 712C will drive the push rod 713 to move with a rotation of the second gear 712, during which the contact surface between the second boss 712C and the elongated hole 713A is constantly changed with the rotation of the second gear 712, i.e., a rolling friction is formed, and the friction force of a rolling friction is significantly less than that of a sliding friction. Therefore, the requirements on the surface roughness and strength of the second boss 712C and the elongated hole 713A can be reduced, thereby simplifying the production process and reducing the production cost. Likewise, during the above process, the acting force on the push rod 713 is also gradually decreased, so that the energy loss of the anvil 120 in the closing operation is reduced, and the efficiency thus is improved. Further details regarding the above operation will be described in further details below.

Reference is now made to Figs. 2, 3 and 9, which illustrate further details of the components included in the second driving assembly 720 more specifically. The second driving assembly 720 includes a rack 721, a third gear 722 and a fourth gear 723.

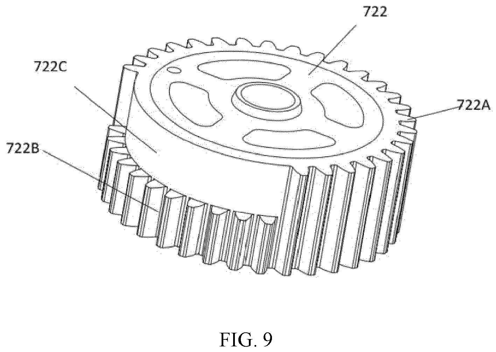

As best illustrated in Fig. 9, the third gear 722 includes a first toothed portion 722A, a second toothed portion 722B and a toothless portion 722C adjacent to the second toothed portion 722B in an up-down direction, which are arranged in a circumference direction of the third gear 722. The third gear 722 is always engaged with the driving gear 731 through the first toothed portion 722A and the second toothed portion 722B. The first toothed portion 722A, the second toothed portion 722B and the toothless portion 722C are cooperated with the fourth gear 723 to form an idling mechanism, the specific principle of which will be described in further details below.

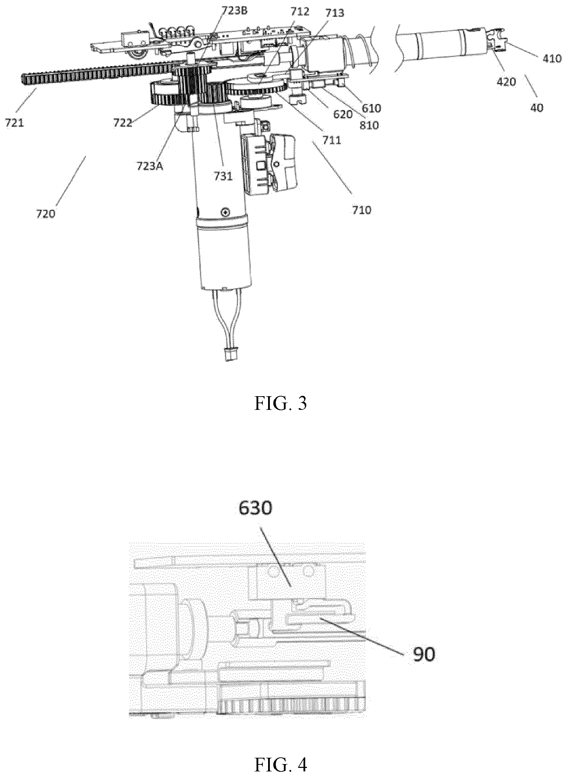

As illustrated in Fig. 3, the fourth gear 723 includes a first sub-gear 723A and a second sub-gear 723B, which have different diameters. The first sub-gear 723A and the second sub-gear 723B may rotate synchronously; and in some embodiments, the first sub-gear 723A and the second sub-gear 723B may be integrally formed. With the rotation, the first sub-gear 723A may be sequentially joined with the first toothed portion 722A and the toothless portion 722C of the third gear 722. The second sub-gear 723B is configured to be engaged with the rack 721. The rack 721 is engaged with the second sub-gear 723B, and one end of the rack 721 is connected with the central spindle 30.

Fig. 3 also illustrates a cutter assembly 40 configured to cut off a tissue of a patient. The cutter assembly 40 includes a cutting knife 410 and a pusher bar 420 connected with the cutting knife 410. A part of the pusher bar 420 is located inside the sleeve 210 and connected with a distal end of the central spindle 30, and the other part of the pusher bar 420 extends into the end effector 10 and is connected with the cutting knife 410. In the illustrated embodiment, the pusher bar 420 includes a concave portion so as to be cooperated with a convex portion of the cutting knife 411, thereby assembling the pusher bar 420 with the cutting knife 411. Of course, other alternative forms can also be envisaged, for example, the convex portion may be provided on the pusher bar 420 while the concave portion may be provided on the cutting knife 411, or the like.

In the embodiment of the present disclosure, when the rack 721 moves in the proximal direction, the central spindle 30 will drive the cutter assembly 40 to move in the proximal direction, thus realizing a retracting operation. In the opposite case, when the rack 721 moves in the distal direction, the central spindle 30 will drive the cutter assembly 40 to move in the distal direction, thus realizing a firing operation. Further details regarding the above operations will be described in further details below.

In the electric stapling instrument according to the present disclosure, a first driving assembly configured to drive at least one of the anvil and the staple cartridge of the end effector to be opened and closed is provided. The first driving assembly is compact in structure and reasonable in design, and can effectively drive at least one of the anvil and the staple cartridge of the end effector to be opened and closed.

In the electric stapling instrument according to the present disclosure, during a movement of the second boss from the third position to the fourth position, a friction force between the second boss and the elongated hole is gradually decreased. Therefore, the requirements on the surface roughness and strength of the second boss and the elongated hole can be reduced, thereby simplifying the production process and reducing the production cost. Similarly, during the above-mentioned process, an acting force on the push rod is also gradually decreased, so that an energy loss of the anvil during a closing operation is reduced, and the efficiency thus is improved.

In the electric stapling instrument according to the present disclosure, a second driving assembly configured to drive the cutter assembly to move in the proximal direction and the distal direction is provided. The second drive assembly is compact in structure and reasonable in design, and can effectively drive the cutter assembly to move in the proximal direction and the distal direction. The second driving assembly and the first driving assembly are driven by the same electric motor and the same driving gear without the need of additionally arranged electric motor and driving gear, so that the number of components, the volume and the cost of the whole electric stapling instrument are all reduced

In the electric stapling instrument according to the present disclosure, an idling mechanism is provided in the first driving assembly, so that the anvil is not driven by the first driving assembly during the movement of the cutter assembly in the proximal direction or the distal direction, thereby remaining the anvil in the closed position or the open position.

In the electric stapling instrument according to the present disclosure, a first limit switch and a second limit switch are arranged on the movement path of the sleeve assembly to indicate whether the anvil that is pivoting is being in place. Moreover, triggering signals of the first limit switch and the second limit switch can be further transmitted to a control module of the electric stapling instrument to control an operation of the electric stapling instrument.

In the electric stapling instrument according to the present disclosure, a third limit switch is provided on the movement path of the rack to indicate whether the cutter assembly that is moving is being in place. Moreover, a triggering signal of the third limit switch can be further transmitted to the control module of the electric stapling instrument to control the operation of the electric stapling instrument.

### 2. Exemplary Operation of Electric Stapling instrument

The structures of the electric stapling instrument according to the present disclosure and the basic functions of respective components have been described above, and the mutual cooperation modes and working principles of these components in the corresponding operations of the electric stapling instrument will be further described in more details below.

### 2.1 Closing Operation of Anvil

### 2.1.1 Closing the anvil:

In order to perform a closing operation of the anvil 120, an operator presses the first button 810, for example. A control module of the electric stapling instrument receives a signal sent by pressing the first button 810, and sends an operation instruction to the motor assembly 50. The motor assembly 50 drives the driving gear 731 to rotate in the first direction, then the driving gear 731 drives the second gear 712 to rotate in the second direction, and the first gear 711 also rotates synchronously in the second direction. In the process of rotation, the second boss 712C of the second gear 712 moves from the position A to the position B in the hole 713A of the push rod 713, so that the sleeve holder 220 moves in the distal direction and drives the sleeve 210 to move in the distal direction, and that the distal end portion 210A of the sleeve 210 drives the anvil 120 to pivot downwards so as to be closed.

### 2.1.2 The cutter assembly remains motionless during the closing operation of the anvil:

While the anvil 120 is closed, the driving gear 731 will also drive the third gear 722 to rotate in the second direction. In this process, the toothless portion 722C of the third gear 722 is joined with the first sub-gear 723A. As a result, the rotation of the third gear 722 will not drive the first sub-gear 723A to rotate, and the second sub-gear 723B will not rotate either, so that the central spindle 30 remains motionless, and that the cutter assembly 40 also remains motionless. In this way, the idling mechanism described above is realized here.

### 2.1.3 The anvil is closed in place:

When the sleeve holder 220 travelling in the distal direction reaches a certain position on its traveling path (i.e., a movement path), a bump 810 (illustrated in Fig. 3) on the sleeve holder 220 triggers a first limit switch 610 (also illustrated in Fig. 3), and the control module receives a signal sent from the first limit switch 610 and instructs the motor assembly 50 to stop rotating. At this time, the boss 712C is located at the position B in the elongated hole 713A, and the anvil 120 is closed to be in place, so that the operator can release the first button 810.

### 2.2 Firing operation

### 2.2.1. The cutter assembly feeds the cutting knife:

In order to perform a firing operation to cut off a tissue of a patient, the operator presses the first button 810, for example. The control module receives a signal sent by pressing the first button 810 and sends an operation instruction to the motor assembly 50. The motor assembly 50 continues to drive the driving gear 731 to rotate in the first direction, then the driving gear 731 drives the third gear 722 to rotate in the second direction, and the first toothed portion 722A of the third gear 722 is engaged with the first sub-gear 723A, thereby driving the first sub-gear 723A to rotate in the first direction and driving the second sub-gear 723B to rotate in the first direction. The second sub-gear 723B drives the rack 721 to move in the distal direction, then the rack 721 drives the central spindle 30 to move in the distal direction, then the central spindle 30 drives the pusher bar 420 to move in the distal direction, and then the pusher bar 420 drives the cutting knife 410 to move in the distal direction, so as to realize feeding the cutting knife to cut tissues.

### 2.2.2 The anvil remains closed during the firing operation:

While the cutter assembly feeds the cutting knife, the driving gear 731 will drive the first gear 711 to rotate in the second direction, and the first boss 711A of the first gear 711 will move from the position C to the position D in the arc-shaped groove 712B of the second gear 712, so that the first gear 711 will be idling without driving the second gear 712 to rotate. At the same time, the driving gear 731 is joined with the toothless portion 712D of the second gear 712 without driving the second gear 712 to rotate, either. In this way, the second gear 712 is in motionless, so as not to drive the sleeve holder 220 to move. Therefore, the anvil remains closed during the firing operation. Here, the idling mechanism described above is realized.

### 2.2.3 Feeding in place:

When the rack 721 travelling in the distal direction reaches a certain position on its path, it pushes an actuator 90 to contact and trigger a third limit switch 630 (both of which are illustrated in Fig. 4). The control module receives a signal from the third limit switch 630 and instructs the motor assembly 50 to stop rotating. Then the cutter assembly 40 that moves in the distal direction is being in place, and the cutting of the tissue is completed.

### 2.3 Retracting operation

### 2.3.1. The cutter assembly retracts the cutting knife:

When the cutting of the tissue is completed and the cutting knife is to be retracted, the operator presses the second button 820, for example. The control module receives a signal sent by pressing the second button 820 and instructs the motor assembly 50 to rotate in the second direction. The motor assembly 50 drives the driving gear 731 to rotate in the second direction, then the driving gear 731 drives the third gear 722 to rotate in the first direction, then the first toothed portion 722A of the third gear 722 drives the first sub-gear 723A to rotate in the second direction, and then the first sub-gear 723A drives the second sub-gear 723B to rotate in the second direction, thereby driving the rack 721 to move in the proximal direction. The rack 721 drives the central spindle 30 to move in the proximal direction, then the central spindle 30 drives the pusher bar 420 to move in the proximal direction, and then the pusher bar 420 drives the cutting knife 410 to move in the proximal direction, thereby retracting the cutting knife.

### 2.3.2 The anvil remains closed during the retracting operation:

While the cutter assembly retracts the cutting knife, the driving gear 731 will drive the first gear 711 to rotate in the first direction, and the first boss 711A of the first gear 711 will move from the position D to the position C in the arc-shaped groove 712B of the second gear 712, so that the first gear 711 will be idling without driving the second gear 712 to rotate. At the same time, because the toothless portion 712D of the second gear 712 is joined with the driving gear 731, the driving gear 731 will not drive the second gear 712 to rotate, either. Thus, the second gear 712 is in motionless without driving the sleeve holder 220 to move. In this way, the anvil remains closed during the retracting operation. The idling mechanism described above is realized here.

### 2.3.3 Retracting in place:

When the rack 721 travelling in the proximal direction reaches a certain position on its path, it pushes the actuator 90 to be disconnected from the third limit switch 630, which indicates the completion of the retracting operation.

### 2.4 Opening Operation of Anvil

### 2.4.1 Opening the anvil:

After the cutting knife is retracted in place, the motor assembly 50, instead of stopping rotating, can continue to drive the driving gear 731 to rotate in the second direction, then the driving gear 731 drives the first gear 711 to rotate in the first direction, and then the first boss 711A of the first gear 711 abuts against an edge of the arc-shaped groove 712B of the second gear 712 so as to drive the second gear 712 to also rotate synchronously in the first direction and to allow the second gear 712 to start engaging with the driving gear 731. During the rotation of the second gear 712, the second boss 712C moves from the position B to the position A in the hole 713A of the push rod 713, so that the push rod 713 drives the sleeve holder 220 to move in the proximal direction. In this process, the anvil 220 pivots upwards so as to be opened.

### 2.4.2 The cutter assembly remains motionless during the opening operation of the anvil:

While the anvil is opened, the driving gear 731 will drive the third gear 722 to rotate in the first direction, and the first toothed portion 722A of the third gear 722 will be disengaged from the first sub-gear 723A, i.e., the toothless portion 722C of the third gear 722 will be joined with the first sub-gear 723A, so as not to drive the first sub-gear 723A to rotate, and the second sub-gear 723B will not rotate, either. Thus, the central spindle 30 will remain motionless. Here, the idling mechanism described above is realized.

### 2.4.3 The anvil is opened in place:

When the sleeve holder 220 retreats to a certain position, the bump 810 triggers the second limit switch 620, and the control module receives a signal from the second limit switch 620 and instructs the motor assembly 50 to stop rotating. At this time, the second boss712C is located at the position A in the elongated hole 713A, and the anvil is opened in place.

It should be understood that the above-mentioned operation procedures, especially the operations of buttons, the working logics of the control module, the setting modes of the limit switches, etc., are only exemplary and are not intended to limit the present disclosure. On the basis of the structure(s) and working principle(s) of the electric stapling instrument according to the present disclosure, those skilled in the art can conceive of other equivalent operation procedures.

Although the present disclosure has been described with reference to the above embodiments, those skilled in the art will understand that various changes can be made without departing from the concept and scope of the present disclosure as defined in the appended claims. Although the specification contains many specific implementation details, these should not be construed as limitations on the scope of the present disclosure, but as descriptions of features specific to particular embodiments. The scope of the present disclosure is defined by the appended claims and their equivalents, and is not limited to the previously described embodiments.

## Claims

1. An electric stapling instrument (1), comprising:
a motor assembly (50) comprising an output shaft configured to rotate in a first direction or a second direction;
a transmission mechanism (70) comprising a driving gear (731) and a first driving assembly (710), the driving gear (731) being installed on the output shaft of the motor assembly (50) and being configured to rotate with the output shaft, wherein the first driving assembly (710) comprises:
a first gear (711) engaged with the driving gear (731) and configured to rotate with the driving gear (731);
a second gear (712) coaxially arranged with the first gear (711), the second gear (712) being at least partially engaged with the driving gear (731) and configured to rotate with the driving gear (731); and
a push rod (713) movably connected with the second gear (712) and configured to move in a proximal direction or a distal direction along with a rotation of the second gear (712);
a sleeve assembly (20) having a proximal end connected with the push rod (713), the sleeve assembly (20) being configured to move in the proximal direction or the distal direction along with the push rod (713); and
an end effector (10) arranged at a distal end of the sleeve assembly (20), wherein the end effector (10) comprises an anvil (120) and a staple cartridge (110), at least one of the anvil (120) and the staple cartridge (110) is configured to be pivotable between an open position and a closed position,
**characterized in that**:
the second gear (712) comprises a second boss (712C), and the push rod (713) comprises an elongated hole (713A) configured to receive the second boss (712C), wherein the second boss (712C) is configured to move between a third position (A) and a fourth position (B) in the elongated hole (713A) so that the push rod (713) moves in the proximal direction or the distal direction along with a rotation of the second gear (712).

2. The electric stapling instrument (1) according to claim 1, wherein the first gear (711) comprises a first boss (711A) and the second gear (712) comprises a groove (712B) configured to receive the first boss (711A), wherein the first boss (711A) is configured to move between a first position (C) and a second position (D) in the groove (712B).

3. The electric stapling instrument (1) according to claim 1, wherein the transmission mechanism (70) further comprises a second driving assembly (720), and the second driving assembly (720) comprises:
a third gear (722) engaged with the driving gear (731) and configured to rotate with the driving gear (731),
a fourth gear (723) engaged with the third gear (722) and arranged to rotate with the third gear (722), and
a rack (721) engaged with the fourth gear (723) and configured to move in the proximal direction or the distal direction along with a rotation of the fourth gear (723); and wherein
the electric stapling instrument (1) further comprises a cutter assembly (40) and a central spindle (30) arranged in the sleeve assembly (20), wherein a proximal end of the central spindle (30) is connected with the rack (721), and the central spindle (30) is configured to move in the proximal direction or the distal direction along with the rack (721); and wherein a proximal end of the cutter assembly (40) is connected with a distal end of the central spindle (30), and the cutter assembly (40) is configured to move in the proximal direction or the distal direction along with the central spindle (30).

4. The electric stapling instrument (1) according to claim 1, wherein the anvil (120) and the staple cartridge (110) are connected with a distal end of the sleeve assembly (20), respectively, and at least one of the anvil (120) and the staple cartridge (110) is configured to pivot between the open position and the closed position in such a manner that:
when the sleeve assembly (20) moves in the proximal direction, at least one of the anvil (120) and the staple cartridge (110) pivots towards the open position; and
when the sleeve assembly (20) moves in the distal direction, at least one of the anvil (120) and the staple cartridge (110) pivots towards the closed position.

5. The electric stapling instrument (1) according to claim 1, wherein,
when the output shaft of the motor assembly (50) rotates in the first direction and drives the driving gear (731) to rotate in the first direction, both the first gear (711) and the second gear (712) rotate with the driving gear (731) in the second direction, so that the second boss (712C) moves from the third position (A) to the fourth position (B) in the elongated hole (713A), causing the push rod (713) to move in the distal direction, and in turn causing the sleeve assembly (20) to move in the distal direction, thereby allowing at least one of the anvil (120) and the staple cartridge (110) to pivot from the open position to the closed position;
when the output shaft of the motor assembly (50) rotates in the second direction and drives the driving gear (731) to rotate in the second direction, both the first gear (711) and the second gear (712) rotate with the driving gear (731) in the first direction, so that the second boss (712C) moves from the fourth position (B) to the third position (A) in the elongated hole (713A), causing the push rod (713) to move in the proximal direction, and in turn causing the sleeve assembly (20) to move in the proximal direction, thereby allowing at least one of the anvil (120) and the staple cartridge (110) to pivot from the closed position to the open position.

6. The electric stapling instrument (1) according to claim 2, wherein the second gear (712) further comprises a toothless portion (722C) and a toothed portion (722B), and a distal end of the electric stapling instrument (1) is further provided with a cutter assembly (40), wherein the cutter assembly (40) is connected with the transmission mechanism (70) and configured to move in the proximal direction or the distal direction, and
when the cutter assembly (40) moves in the proximal direction or the distal direction, the first driving assembly (710) is further configured such that:
when the first gear (711) rotates, the first boss (711A) moves from the first position (C) to the second position (D) or moves from the second position (D) to the first position (C) in the groove (712B), so that the first gear (711) idles relative to the second gear (712) without driving the second gear (712) to rotate; and,
the toothless portion (722C) of the second gear (712) is joined with the driving gear (731) so as not to rotate the second gear (712) with the driving gear (731), so that the sleeve assembly (20) remains motionless, thereby allowing at least one of the anvil (120) and the staple cartridge (110) to remain in the closed position or the open position.

7. The electric stapling instrument (1) according to any one of claims 1 to 6, further comprising a first limit switch (610) arranged on a movement path of the sleeve assembly (20), wherein
the motor assembly (50) is configured to stop rotating when the sleeve assembly (20) traveling in the distal direction contacts and triggers the first limit switch (610), so that at least one of the anvil (120) and the staple cartridge (110) is in the closed position.

8. The electric stapling instrument (1) according to any one of claims 1 to 6, further comprising a second limit switch (620) arranged on a movement path of the sleeve assembly (20),
wherein the motor assembly (50) is configured to stop rotating when the sleeve assembly (20) travelling in the proximal direction contacts and triggers the second limit switch (620), so that at least one of the anvil (120) and the staple cartridge (110) is in the open position.

9. The electric stapling instrument (1) according to claim 4, further comprising a third limit switch (630) arranged on a movement path of the rack (721),
wherein the motor assembly (50) is configured to stop rotating when the rack (721) travelling in the distal direction contacts and triggers the third limit switch (630), so that the cutter assembly (40) is in place by moving in the distal direction.

## Patentansprüche

1. Elektrisches Klammergerät (1), aufweisend:
eine Motoreinheit (50), die eine Ausgangswelle aufweist, die dazu eingerichtet ist, sich in einer ersten Richtung oder einer zweiten Richtung zu drehen;
einen Getriebemechanismus (70), der ein Antriebszahnrad (731) und eine erste Antriebseinheit (710) aufweist, wobei das Antriebszahnrad (731) an der Ausgangswelle der Motoreinheit (50) angebracht und dazu eingerichtet ist, sich mit der Ausgangswelle zu drehen, wobei die erste Antriebseinheit (710) aufweist:
ein erstes Zahnrad (711), das mit dem Antriebszahnrad (731) in Eingriff steht und dazu eingerichtet ist, sich mit dem Antriebszahnrad (731) zu drehen;
ein zweites Zahnrad (712), das koaxial zu dem ersten Zahnrad (711) angeordnet ist, wobei das zweite Zahnrad (712) zumindest teilweise mit dem Antriebszahnrad (731) in Eingriff steht und dazu eingerichtet ist, sich mit dem Antriebszahnrad (731) zu drehen; und
eine Schubstange (713), die beweglich mit dem zweiten Zahnrad (712) verbunden und dazu eingerichtet ist, sich bei einer Drehung des zweiten Zahnrads (712) in einer proximalen Richtung oder einer distalen Richtung zu bewegen;
eine Hülseneinheit (20), die ein mit der Schubstange (713) verbundenes proximales Ende aufweist, wobei die Hülsenanordnung (20) dazu eingerichtet ist, sich zusammen mit der Schubstange (713) in der proximalen Richtung oder der distalen Richtung zu bewegen; und
einen Endeffektor (10), der an einem distalen Ende der Hülseneinheit (20) angeordnet ist, wobei der Endeffektor (10) einen Amboss (120) und ein Klammermagazin (110) aufweist, wobei mindestens eines von dem Amboss (120) und dem Klammermagazin (110) dazu eingerichtet ist, zwischen einer offenen Position und einer geschlossenen Position schwenkbar zu sein,
**dadurch gekennzeichnet, dass**:
das zweite Zahnrad (712) einen zweiten Vorsprung (712C) aufweist und die Schubstange (713) ein Langloch (713A) aufweist, das dazu eingerichtet ist, den zweiten Vorsprung (712C) aufzunehmen, wobei der zweite Vorsprung (712C) dazu eingerichtet ist, sich zwischen einer dritten Position (A) und einer vierten Position (B) in dem Langloch (713A) zu bewegen, sodass sich die Schubstange (713) bei einer Drehung des zweiten Zahnrads (712) in der proximalen Richtung oder der distalen Richtung bewegt.

2. Elektrisches Klammergerät (1) nach Anspruch 1, wobei das erste Zahnrad (711) einen ersten Vorsprung (711A) aufweist und das zweite Zahnrad (712) eine Nut (712B) aufweist, die zur Aufnahme des ersten Vorsprungs eingerichtet ist, wobei der erste Vorsprung (711A) so eingerichtet ist, sich zwischen einer ersten Position (C) und einer zweiten Position (D) in der Nut (712B) zu bewegen.

3. Elektrisches Klammergerät (1) nach Anspruch 1, wobei der Getriebemechanismus (70) ferner eine zweite Antriebseinheit (720) aufweist und die zweite Antriebseinheit (720) aufweist:
ein drittes Zahnrad (722), das mit dem Antriebszahnrad (731) in Eingriff steht und dazu eingerichtet ist, sich mit dem Antriebszahnrad (731) zu drehen,
ein viertes Zahnrad (723), das mit dem dritten Zahnrad (722) in Eingriff steht und dazu angeordnet ist, sich mit dem dritten Zahnrad (722) zu drehen, und
eine Zahnstange (721), die mit dem vierten Zahnrad (723) in Eingriff steht und dazu eingerichtet ist, sich bei einer Drehung des vierten Zahnrads (723) in der proximalen Richtung oder der distalen Richtung zu bewegen; und wobei
das elektrische Klammergerät (1) ferner eine Schneideinheit (40) und eine zentrale Spindel (30) aufweist, die in der Hülseneinheit (20) angeordnet sind, wobei ein proximales Ende der zentralen Spindel (30) mit der Zahnstange (721) verbunden ist und die zentrale Spindel (30) dazu eingerichtet ist, sich zusammen mit der Zahnstange (721) in der proximalen Richtung oder der distalen Richtung zu bewegen; und wobei ein proximales Ende der Schneideinheit (40) mit einem distalen Ende der zentralen Spindel (30) verbunden ist und die Schneideinheit (40) dazu eingerichtet ist, sich zusammen mit der zentralen Spindel (30) in der proximalen Richtung oder der distalen Richtung zu bewegen.

4. Elektrisches Klammergerät (1) nach Anspruch 1, wobei der Amboss (120) und das Klammermagazin (110) jeweils mit einem distalen Ende der Hülseneinheit (20) verbunden sind und mindestens eines von dem Amboss (120) und dem Klammermagazin (110) dazu eingerichtet ist, zwischen der offenen Position und der geschlossenen Position derart zu schwenken, dass:
wenn sich die Hülseneinheit (20) in der proximalen Richtung bewegt, mindestens eines von dem Amboss (120) und dem Klammermagazin (110) in Richtung der offenen Position schwenkt; und wenn sich die Hülseneinheit (20) in der distalen Richtung bewegt, mindestens eines von dem Amboss (120) und dem Klammermagazin (110) in Richtung der geschlossenen Position schwenkt.

5. Elektrisches Klammergerät (1) nach Anspruch 1, wobei,
wenn sich die Ausgangswelle der Motoreinheit (50) in der ersten Richtung dreht und dabei das Antriebszahnrad (731) in der ersten Richtung antreibt, sich sowohl das erste Zahnrad (711) als auch das zweite Zahnrad (712) mit dem Antriebszahnrad (731) in der zweiten Richtung drehen, sodass sich der zweite Vorsprung (712C) von der dritten Position (A) zu der vierten Position (B) in dem Langloch (713A) bewegt, wodurch sich die Schubstange (713) in der distalen Richtung bewegt und dadurch die Hülseneinheit (20) in der distalen Richtung bewegt, sodass mindestens eines von dem Amboss (120) und dem Klammermagazin (110) von der offenen Position in die geschlossene Position schwenkt;
wenn sich die Ausgangswelle der Motoreinheit (50) in der zweiten Richtung dreht und dabei das Antriebszahnrad (731) in der zweiten Richtung antreibt, sich sowohl das erste Zahnrad (711) als auch das zweite Zahnrad (712) mit dem Antriebszahnrad (731) in der ersten Richtung drehen, sodass sich der zweite Vorsprung (712C) von der vierten Position (B) zu der dritten Position (A) in dem Langloch (713A) bewegt, wodurch sich die Schubstange (713) in der proximalen Richtung bewegt und dadurch die Hülseneinheit (20) in der proximalen Richtung bewegt, sodass mindestens eines von dem Amboss (120) und dem Klammermagazin (110) von der geschlossenen Position in die offene Position schwenkt.

6. Elektrisches Klammergerät (1) nach Anspruch 2, wobei das zweite Zahnrad (712) ferner einen zahnlosen Abschnitt (722C) und einen verzahnten Abschnitt (722B) aufweist und ein distales Ende des elektrischen Klammergeräts (1) ferner mit einer Schneideinheit (40) versehen ist, wobei die Schneideinheit (40) mit dem Getriebemechanismus (70) verbunden und dazu eingerichtet ist, sich in der proximalen Richtung oder der distalen Richtung zu bewegen, und
wenn sich die Schneideinheit (40) in der proximalen Richtung oder der distalen Richtung bewegt, die erste Antriebseinheit (710) ferner derart eingerichtet ist, dass:
wenn sich das erste Zahnrad (711) dreht, sich der erste Vorsprung (711A) von der ersten Position (C) zu der zweiten Position (D) oder von der zweiten Position (D) zu der ersten Position (C) in der Nut (712B) bewegt, sodass sich das erste Zahnrad (711) relativ zu dem zweiten Zahnrad (712) im Leerlauf dreht, ohne das zweite Zahnrad (712) zur Drehung anzutreiben; und
der zahnlose Abschnitt (722C) des zweiten Zahnrads (712) mit dem Antriebszahnrad (731) in Kontakt steht, sodass sich das zweite Zahnrad (712) nicht mit dem Antriebszahnrad (731) dreht und die Hülseneinheit (20) somit bewegungslos bleibt, wobei mindestens eines von dem Amboss (120) und dem Klammermagazin (110) in der geschlossenen Position oder der offenen Position bleibt.

7. Elektrisches Klammergerät (1) nach einem der Ansprüche 1 bis 6, ferner aufweisend einen ersten Endschalter (610), der auf einem Bewegungspfad der Hülseneinheit (20) angeordnet ist, wobei
die Motoreinheit (50) dazu eingerichtet ist, die Drehung zu stoppen, wenn die sich in der distalen Richtung bewegende Hülseneinheit (20) den ersten Endschalter (610) berührt und auslöst, sodass sich mindestens eines von dem Amboss (120) und dem Klammermagazin (110) in der geschlossenen Position befindet.

8. Elektrisches Klammergerät (1) nach einem der Ansprüche 1 bis 6, ferner aufweisend einen zweiten Endschalter (620), der auf einem Bewegungspfad der Hülseneinheit (20) angeordnet ist,
wobei die Motoreinheit (50) dazu eingerichtet ist, die Drehung zu stoppen, wenn die sich in der proximalen Richtung bewegende Hülseneinheit (20) den zweiten Endschalter (620) berührt und auslöst, sodass sich mindestens eines von dem Amboss (120) und dem Klammermagazin (110) in der offenen Position befindet.

9. Elektrisches Klammergerät (1) nach Anspruch 4, ferner aufweisend einen dritten Endschalter (630), der auf einem Bewegungspfad der Zahnstange (721) angeordnet ist, wobei die Motoreinheit (50) dazu eingerichtet ist, die Drehung zu stoppen, wenn die sich in der distalen Richtung bewegende Zahnstange (721) den dritten Endschalter (630) berührt und auslöst, sodass die Schneideinheit (40) durch eine Bewegung in der distalen Richtung in Position gelangt.

## Revendications

1. Instrument d'agrafage électrique (1), comprenant :
un ensemble moteur (50) comprenant un arbre de sortie configuré pour tourner dans une première direction ou une seconde direction ;
un mécanisme de transmission (70) comprenant un engrenage d'entraînement (731) et un premier ensemble d'entraînement (710), l'engrenage d'entraînement (731) étant installé sur l'arbre de sortie de l'ensemble moteur (50) et étant configuré pour tourner avec l'arbre de sortie, dans lequel le premier ensemble d'entraînement (710) comprend :
un premier engrenage (711) en prise avec l'engrenage d'entraînement (731) et configuré pour tourner avec l'engrenage d'entraînement (731) ;
un second engrenage (712) disposé coaxialement au premier engrenage (711), le second engrenage (712) étant au moins partiellement en prise avec l'engrenage d'entraînement (731) et configuré pour tourner avec l'engrenage d'entraînement (731) ; et
une tige de poussée (713) reliée de manière mobile au second engrenage (712) et configurée pour se déplacer dans une direction proximale ou une direction distale sous l'effet d'une rotation du second engrenage (712) ;
un ensemble de manchon (20) ayant une extrémité proximale reliée à la tige de poussée (713), l'ensemble de manchon (20) étant configuré pour se déplacer dans la direction proximale ou la direction distale avec la tige de poussée (713) ; et
un effecteur terminal (10) disposé à une extrémité distale de l'ensemble de manchon (20), dans lequel l'effecteur terminal (10) comprend une enclume (120) et une cartouche d'agrafes (110), au moins l'une parmi l'enclume (120) et la cartouche d'agrafes (110) étant configurée pour pouvoir pivoter entre une position ouverte et une position fermée,
**caractérisé en ce que** :
le second engrenage (712) comprend un second bossage (712C), et la tige de poussée (713) comprend un trou allongé (713A) configuré pour recevoir le second bossage (712C), dans lequel le second bossage (712C) est configuré pour se déplacer entre une troisième position (A) et une quatrième position (B) dans le trou allongé (713A), de sorte que la tige de poussée (713) se déplace dans la direction proximale ou la direction distale sous l'effet d'une rotation du second engrenage (712).

2. Instrument d'agrafage électrique (1) selon la revendication 1, dans lequel le premier engrenage (711) comprend un premier bossage (711A) et le second engrenage (712) comprend une rainure (712B) configurée pour recevoir le premier bossage (711A), dans lequel le premier bossage (711A) est configuré pour se déplacer entre une première position (C) et une deuxième position (D) dans la rainure (712B).

3. Instrument d'agrafage électrique (1) selon la revendication 1, dans lequel le mécanisme de transmission (70) comprend en outre un deuxième ensemble d'entraînement (720), et le deuxième ensemble d'entraînement (720) comprend :
un troisième engrenage (722) en prise avec l'engrenage d'entraînement (731) et configuré pour tourner avec l'engrenage d'entraînement (731),
un quatrième engrenage (723) en prise avec le troisième engrenage (722) et disposé pour tourner avec le troisième engrenage (722), et
une crémaillère (721) en prise avec le quatrième engrenage (723) et configurée pour se déplacer dans la direction proximale ou la direction distale sous l'effet d'une rotation du quatrième engrenage (723) ; et dans lequel
l'instrument d'agrafage électrique (1) comprend en outre un ensemble de coupe (40) et une broche centrale (30) disposée dans l'ensemble de manchon (20), dans lequel une extrémité proximale de la broche centrale (30) est reliée à la crémaillère (721), et la broche centrale (30) est configurée pour se déplacer dans la direction proximale ou la direction distale avec la crémaillère (721) ; et dans lequel une extrémité proximale de l'ensemble de coupe (40) est reliée à une extrémité distale de la broche centrale (30), et l'ensemble de coupe (40) est configuré pour se déplacer dans la direction proximale ou la direction distale avec la broche centrale (30).

4. Instrument d'agrafage électrique (1) selon la revendication 1, dans lequel l'enclume (120) et la cartouche d'agrafes (110) sont respectivement reliées à une extrémité distale de l'ensemble de manchon (20), et au moins l'une parmi l'enclume (120) et la cartouche d'agrafes (110) est configurée pour pivoter entre la position ouverte et la position fermée de telle manière que :
lorsque l'ensemble de manchon (20) se déplace dans la direction proximale, au moins l'une parmi l'enclume (120) et la cartouche d'agrafes (110) pivote vers la position ouverte ; et lorsque l'ensemble de manchon (20) se déplace dans la direction distale, au moins l'une parmi l'enclume (120) et la cartouche d'agrafes (110) pivote vers la position fermée.

5. Instrument d'agrafage électrique (1) selon la revendication 1, dans lequel,
lorsque l'arbre de sortie de l'ensemble moteur (50) tourne dans la première direction et entraîne l'engrenage d'entraînement (731) à tourner dans la première direction, le premier engrenage (711) et le second engrenage (712) tournent tous deux avec l'engrenage d'entraînement (731) dans la seconde direction, de sorte que le second bossage (712C) se déplace de la troisième position (A) à la quatrième position (B) dans le trou allongé (713A), ce qui provoque le déplacement de la tige de poussée (713) dans la direction distale et entraîne à son tour le déplacement de l'ensemble de manchon (20) dans la direction distale, permettant ainsi à au moins l'une parmi l'enclume (120) et la cartouche d'agrafes (110) de pivoter de la position ouverte à la position fermée ;
lorsque l'arbre de sortie de l'ensemble moteur (50) tourne dans la seconde direction et entraîne l'engrenage d'entraînement (731) à tourner dans la seconde direction, le premier engrenage (711) et le second engrenage (712) tournent tous deux avec l'engrenage d'entraînement (731) dans la première direction, de sorte que le second bossage (712C) se déplace de la quatrième position (B) à la troisième position (A) dans le trou allongé (713A), ce qui provoque le déplacement de la tige de poussée (713) dans la direction proximale et entraîne à son tour le déplacement de l'ensemble de manchon (20) dans la direction proximale, permettant ainsi à au moins l'une parmi l'enclume (120) et la cartouche d'agrafes (110) de pivoter de la position fermée à la position ouverte.

6. Instrument d'agrafage électrique (1) selon la revendication 2, dans lequel le second engrenage (712) comprend en outre une partie non dentée (722C) et une partie dentée (722B), et une extrémité distale de l'instrument d'agrafage électrique (1) est en outre pourvue d'un ensemble de coupe (40), dans lequel l'ensemble de coupe (40) est relié au mécanisme de transmission (70) et configuré pour se déplacer dans la direction proximale ou la direction distale, et
lorsque l'ensemble de coupe (40) se déplace dans la direction proximale ou dans la direction distale, le premier ensemble d'entraînement (710) est en outre configuré de telle sorte que :
lorsque le premier engrenage (711) tourne, le premier bossage (711A) se déplace de la première position (C) à la deuxième position (D) ou se déplace de la deuxième position (D) à la première position (C) dans la rainure (712B), de sorte que le premier engrenage (711) tourne à vide par rapport au second engrenage (712) sans entraîner le second engrenage (712) à tourner; et
la partie non dentée (722C) du second engrenage (712) est en contact avec l'engrenage d'entraînement (731) de manière à ne pas faire tourner le second engrenage (712) avec l'engrenage d'entraînement (731), de sorte que l'ensemble de manchon (20) reste immobile, permettant ainsi à au moins l'une parmi l'enclume (120) et la cartouche d'agrafes (110) de rester en position fermée ou en position ouverte.

7. Instrument d'agrafage électrique (1) selon l'une quelconque des revendications 1 à 6, comprenant en outre un premier interrupteur de fin de course (610) disposé sur une trajectoire de déplacement de l'ensemble de manchon (20), dans lequel
l'ensemble moteur (50) est configuré pour arrêter de tourner lorsque l'ensemble de manchon (20) se déplaçant dans la direction distale entre en contact avec et déclenche le premier interrupteur de fin de course (610), de sorte qu'au moins l'une parmi l'enclume (120) et la cartouche d'agrafes (110) se trouve dans la position fermée.

8. Instrument d'agrafage électrique (1) selon l'une quelconque des revendications 1 à 6, comprenant en outre un deuxième interrupteur de fin de course (620) disposé sur une trajectoire de déplacement de l'ensemble de manchon (20),
dans lequel l'ensemble moteur (50) est configuré pour arrêter de tourner lorsque l'ensemble de manchon (20) se déplaçant dans la direction proximale entre en contact avec et déclenche le deuxième interrupteur de fin de course (620), de sorte qu'au moins l'une parmi l'enclume (120) et la cartouche d'agrafes (110) se trouve dans la position ouverte.

9. Instrument d'agrafage électrique (1) selon la revendication 4, comprenant en outre un troisième interrupteur de fin de course (630) disposé sur une trajectoire de déplacement de la crémaillère (721), dans lequel l'ensemble moteur (50) est configuré pour arrêter de tourner lorsque la crémaillère (721) se déplaçant dans la direction distale entre en contact avec et déclenche le troisième interrupteur de fin de course (630), de sorte que l'ensemble de coupe (40) se mette en place en se déplaçant dans la direction distale.
